Europäisches Patentamt

European Patent Office

Office européen des brevets

(19)

(11)  **EP 1 179 598 B1**

(12)  **EUROPEAN PATENT SPECIFICATION**

(45) Date of publication and mention
of the grant of the patent:
**10.08.2005  Bulletin 2005/32**

(21) Application number: **00927766.6**

(22) Date of filing: **15.05.2000**

(51) Int Cl.$^7$: **C12P 19/38**

(86) International application number:
**PCT/JP2000/003095**

(87) International publication number:
**WO 2000/070074 (23.11.2000 Gazette 2000/47)**

(54) **PROCESS FOR PRODUCING NUCLEOSIDE COMPOUND**

VERFAHREN ZUR HERSTELLUNG VON NUKLEOSID-VERBINDUNG

PROCEDE DE PRODUCTION DE COMPOSE NUCLEOSIDIQUE

(84) Designated Contracting States:
**AT BE CH CY DE DK ES FI FR GB GR IE IT LI LU
MC NL PT SE**

(30) Priority: **13.05.1999  JP 13214499**

(43) Date of publication of application:
**13.02.2002  Bulletin 2002/07**

(73) Proprietor: **MITSUI CHEMICALS, INC.
Tokyo (JP)**

(72) Inventors:
• **NIKUMARU, Seiya
Mobara-shi, Chiba 297-0017 (JP)**
• **NAKAMURA, Takeshi
Ichihara-shi, Chiba 290-0158 (JP)**

(74) Representative: **Kirkham, Nicholas Andrew et al
Graham Watt & Co LLP
St Botolph's House
7-9 St Botolph's Road
Sevenoaks Kent TN13 3AJ (GB)**

(56) References cited:
**EP-A2- 0 198 387         JP-A- 1 104 190
JP-A- 11 137 290         JP-A- 59 213 397**

• **OUWERKERK N ET AL: "One-pot two-step
enzymatic coupling of pyrimidine bases to
2-deoxy-D-ribose-5-phosphate. A new strategy
in the synthesis of stable isotope labeled
deoxynucleosides." JOURNAL OF ORGANIC
CHEMISTRY, vol. 67, no. 5, 8 March 2002
(2002-03-08), pages 1480-1489, XP002220339
March 8, 2002 ISSN: 0022-3263**

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

EP 1 179 598 B1

**Description**

Technical Field

This invention relates to a process for preparing a nucleoside compound using a nucleoside phosphorylase. A variety of nucleosides and their analogues are used as a starting material for synthesis or drug formulation in production of an antiviral, anticancer or antisense agent.

Background Art

The term "nucleoside phosphorylase" is a generic name for enzymes involved in phosphorolysis of an N-glycoside bond in a nucleoside in the presence of phosphoric acid. As an example using ribonucleoside, it catalyzes a reaction represented by the following formula:

Ribonucleoside + Phosphoric acid (phosphate) $\rightarrow$

Nucleic acid base + Ribose-1-phosphate

The enzymes, which may be generally classified into purine nucleoside phosphorylases and pyrimidine nucleoside phosphorylases, are widely distributed in the living world; for example, they are present in tissues of a mammal, bird or fish; yeasts; and bacteria. The enzyme reaction is reversible and synthesis of various nucleoside compounds using the reverse reaction is known several years past.

For example, there is known a process for producing thymidine (JP-A 01-104190), 2'-deoxyadenosine (JP-A 11-137290) or 2'-deoxyguanosine (JP-A 11-137290) from 2'-deoxyribose-1-phosphate and a nucleic acid base (thymine, adenine or guanine, respectively). In addition, Agric. Biol. Chem., Vol. 50 (1), pp. 121-126 (1986) describes a technique to produce ribavirin, as an antiviral agent by decomposing inosine into ribose-1-phosphate and hypoxanthine as a reaction using a purine nucleoside phosphorylase derived from Enterobacter aerogenes in the presence of phosphoric acid and reacting ribose-1-phosphate isolated by an ion-exchange resin and 1,2,4-triazole-3-carboxamide also using purine nucleoside phosphorylase derived from Enterobacter aerogenes.

However, since the reaction for forming the nucleoside compound from a pentose-1-phosphate or its salt and a nucleic acid base by the reverse reaction using the enzyme is an equilibrium reaction, there has been a technical drawback that a conversion rate could not be improved.

When a nucleoside is industrially produced, it is essential to proceed with this reaction wit a higher conversion rate in order to reduce a raw material cost and improve purity of the product thus obtained.

Thus, an object of this invention is to provide a process which is more widely applicable for preparing a nucleoside compound comprising the step of reacting a pentose-1-phosphate with a nucleic acid base or its analogue in the presence of nucleoside phosphorylase activity with an improved rate of conversion into the nucleoside compound though the reaction.

Disclosure of the Invention

After intensive investigation for solving the these problems, we have found that the conversion rate can be improved by allowing a metal cation capable of forming a water-insoluble salt with a phosphate ion to be present in a reaction solution, because the reaction equilibrium sifts toward the direction for synthesizing the nucleoside compound by removing the phosphate ion, as a byproduct of the reaction, from the reaction system as a water-insoluble salt precipitate.

We have further found that almost the same effect can be obtained by the presence of the metal cation capable of forming the water-insoluble salt with phosphoric acid in the form of a pentose-1-phosphate salt in the reaction solution, in comparison with the above addition of the metal cation or its salt capable of forming a water-insoluble salt with phosphoric acid in to the reaction solution.

This invention is based on these findings.

Particularly, a process according to the present invention for preparing a nucleoside compound comprising a step of reacting a pentose-1-phosphate with a nucleic acid base or its analogue in the presence of nucleoside phosphorylase activity in an aqueous reaction medium to form the nucleoside compound, which is characterized in allowing a metal cation capable of forming a water-insoluble salt with phosphate ion to be present in the aqueous reaction medium.

Ribose-1-phosphate or 2-deoxyribose-1-phosphate may be used in this preparation process as the pentose-1-phosphate.

As the metal cation capable of forming a water-insoluble salt with phosphoric acid, at least one selected from the group consisting of calcium, barium and aluminum ions may be used.

The metal cation capable of forming a water-insoluble salt with phosphoric acid may be added to the aqueous reaction medium as a metal salt with at least one anion selected from the group consisting of chloride, nitride, carbonate, sulfate and acetate ions.

The metal cation capable of forming a water-insoluble salt with phosphoric acid may be present as a pentose-1-phosphate salt in the aqueous reaction medium.

According to this invention, a reaction yield can be significantly improved in the synthetic reaction of the nucleoside compound from the pentose-1-phosphate and the nucleic acid base or its analogue using the enzyme activity of the nucleoside phosphorylase in the presence of the metal cation capable of forming a water-insoluble salt with phosphoric acid, and, thus, an effective process for preparing the nucleoside compound can be provided.

Best Mode for Carrying Out the Invention

The term "pentose-1-phosphate" as used herein refers to a polyhydroxy aldehyde or polyhydroxy ketone or its derivative to which a phosphate group is attached at the first position, position 1, via an ester linkage.

Typical examples thereof include, but not limited to, ribose-1-phosphate, 2'-deoxyribose-1-phosphate, 2',3'-dideoxyribose-1-phosphate and arabinose-1-phosphate.

Examples of a polyhydroxy aldehyde or polyhydroxy ketone herein include, but not limited to, aldopentoses such as D-arabinose, L-arabinose, D-xylose, L-lyxose and D-ribose; ketopentoses such as D-xylulose, L-xylulose and D-ribulose; and deoxysaccharides such as D-2-deoxyribose and D-2,3-dideoxyribose, as those from a natural source.

Such a pentose-1-phosphate may be prepared by a known process, including phosphorolysis of a nucleoside compound using activity of a nucleoside phosphorylase as described in J. Biol. Chem. Vol. 184, 437 (1950); and an anomer-selective chemical synthesis.

A nucleic acid base or its analogue used in this invention is a nitrogen-containing heterocyclic compound which is an element for the chemical structural formation of DNA or RNA, including natural or synthetic nucleic acid bases or their analogues selected from the group consisting of pyrimidine, purine and nucleic-acid-base analogues.

Such a nucleic acid base or its analogue may be substituted with a halogen atom(s), or an alkyl group, a haloalkyl group, an alkenyl group, a haloalkenyl group, an alkynyl group, an amino group, an alkylamino group, a hydroxy group, a hydroxyamino group, an aminooxy group, an alkoxy group, a mercapto group, an alkylmercapto group, an aryl group, an aryloxy group or a cyano group.

Examples of the halogen atom as a substituent include chlorine, fluorine, iodine and bromine atoms.

Examples of the alkyl group include an alkyl group having 1 to 7 carbon atoms such as a methyl group, an ethyl group and a propyl group.

Examples of the haloalkyl group include those having an alkyl group having 1 to 7 carbon atoms such as a fluoromethyl group, a difluoromethyl group, a trifluoromethyl group, a bromomethyl group and a bromoethyl group.

Examples of the alkenyl group include those having 2 to 7 carbon atoms such as a vinyl group and an allyl group.

Examples of the haloalkenyl group include those having an alkenyl group having 2 to 7 carbon atoms such as a bromovinyl group and a chlorovinyl group.

Examples of the alkynyl group include those having 2 to 7 carbon atoms such as an ethynyl group and a propynyl group.

Examples of the alkylamino group include those having an alkyl group with 1 to 7 carbon atoms such as a methylamino group and an ethylamino group.

Examples of the alkoxy group include those with 1 to 7 carbon atoms such as a methoxy group and an ethoxy group.

Examples of the alkylmercapto group include those having an alkyl group with 1 to 7 carbon atoms such as a methylmercapto group and an ethylmercapto group.

Examples of the aryl group include a phenyl group; an alkylphenyl group having an alkyl group(s) with 1 to 5 carbon atoms such as a methylphenyl group and an ethylphenyl group; an alkoxyphenyl group having an alkoxy group (s) with 1 to 5 carbon atoms such as a methoxyphenyl group and an ethoxyphenyl group; an alkylaminophenyl group having an alkylamino group(s) with 1 to 5 carbon atoms such as a dimethylaminophenyl group and a diethylaminophenyl group; and a halogenophenyl group such as a chlorophenyl group and a bromophenyl group.

Examples of the pyrimidine compound include cytosine, uracil, 5-fluorocytosine, 5-fluoro uracil, 5-chlorocytosine, 5-chlorouracil, 5-bromocytosine, 5-bromouracil, 5-iodocytosine, 5-iodouracil, 5-methylcytosine, 5-methyluracil (thymine), 5-ethylcytosine, 5-ethyluracil, 5-fluoromethylcytosine, 5-fluorouracil, 5-trifluorocytosine, 5-trifluorouracil, 5-vinyluracil, 5-bromovinyluracil, 5-chlorovinyluracil, 5-ethynylcytosine, 5-ethynyluracil, 5-propynyluracil, pyrimidin-2-one, 4-hydroxyaminopyrimidin-2-one, 4-aminoxypyrimidin-2-one, 4-methoxypyrimidin-2-one, 4-acetoxypyrimidin-2-one, 4-fluoropyrimidin-2-one and 5-fluoropyrimidin-2-one.

Examples of the purine compound include purine, 6-aminopurine (adenine), 6-hydroxypurine, 6-fluoropurine, 6-chloropurine, 6-methylaminopurine, 6-dimethylaminopurine, 6-trifluoromethylaminopurine, 6-benzoylaminopurine, 6-acetylaminopurine, 6-hydroxyaminopurine, 6-aminoxypurine, 6-methoxypurine, 6-acetoxypurine, 6-benzoyloxypurine, 6-methylpurine, 6-ethylpurine, 6-trifluoromethylpurine, 6-phenylpurine, 6-mercaptopurine, 6-methylmercaptopurine, 6-aminopurine-1-oxide, 6-hydroxypurine-1-oxide, 2-amino-6-hydroxypurine (guanine), 2,6-diaminopurine, 2-amino-6-chloropurine, 2-amino-6-iodopurine, 2-aminopurine, 2-amino-6-mercaptopurine, 2-amino-6-methylmercaptopurine, 2-amino-6-hydroxyaminopurine, 2-amino-6-methoxypurine, 2-amino-6-benzoyloxypurine, 2-amino-6-acetoxypurine, 2-amino-6-methylpurine, 2-amino-6-cyclopropylaminomethylpurine, 2-amino-6-phenylpurine, 2-amino-8-bromopurine, 6-cyanopurine, 6-amino-2-chloropurine (2-chloroadenine) and 6-amino-2-fluoropurine (2-fluoroadenine).

Examples of a nucleic acid base analogue include 6-amino-3-deazapurine, 6-amino-8-azapurine, 2-amino-6-hydroxy-8-azapurine, 6-amino-7-deazapurine, 6-amino-1-deazapurine and 6-amino-2-azapurine.

A nucleoside compound in this invention refers to the above polyhydroxy aldehyde or polyhydroxy ketone to which the pyrimidine compound, the purine compound or the nucleic acid base analogue is attached at position 1 via an N-glycoside linkage. Examples include thymidine, deoxythymidine, deoxyuridine, deoxyguanosine, deoxyadenosine, dideoxyadenosine, trifluorothymidine, ribavirin, orotidine, uracil arabinoside, adenine arabinoside, 2-methyl-adenine arabinoside, 2-chloro-hypoxanthine arabinoside, thioguanine arabinoside, 2,6-diaminopurine arabinoside, cytosine arabinoside, guanine arabinoside, thymine arabinoside, enocitabine, gemcitabine, azidothymidine, idoxuridine, dideoxyadenosine, dideoxyinosine, dideoxycytidine, didehydrodeoxythymidine, thiadideoxycytidine, sorivudine, 5-methyluridine, virazole, thioinosine, tegafur, doxifluridine, bredinin, nebularine, allopurinol uracil, 5-fluorouracil, 2'-aminouridine, 2'-aminoadenosine, 2'-aminoguanidine and 2-chloro-2'-aminoinosine.

A nucleoside phosphorylase is a generic name for enzymes capable of phosphorolysis of an N-glycoside bond in a nucleoside in the presence of phosphoric acid, and its reverse reaction can be applied to this invention by using this enzyme activity. An enzyme used in the reaction may be of any type or origin as long as it has an activity of forming a desired nucleoside compound from a corresponding pentose-1-phosphate and a nucleic acid base or its analogue. The enzymes may be generally categorized into two types, purine and pyrimidine types. Known examples of a purine type enzyme include purine nucleoside phosphorylase (EC2.4.2.1) and guanosine phosphorylase (EC2.4.2.15). Examples of a pyrimidine type enzyme include pyrimidine nucleoside phosphorylase (EC2.4.2.2), uridine phosphorylase (EC2.4.2.3), thymidine phosphorylase (EC2.4.2.4) and deoxyuridine phosphorylase (EC2.4.2.23).

Nucleoside phosphorylase activity in this invention may be applied to a reaction system by using a microorganism having the enzyme activity in an aqueous reaction medium or in contact with an aqueous reaction medium. Such a microorganism may be, with no restrictions, any microorganism expressing at least one nucleoside phosphorylase selected from the group consisting of purine nucleoside phosphorylase (EC2.4.2.1), guanosine phosphorylase (EC2.4.2.15), pyrimidine nucleoside phosphorylase (EC2.4.2.2), uridine phosphorylase (EC2.4.2.3), thymidine phosphorylase (EC2.4.2.4) and deoxyuridine phosphorylase (EC2.4.2.23).

Preferable examples of such a microorganism include strains belonging to the genuses Nocardia, Microbacterium, Corynebacterium, Brevibacterium, Cellulomonas, Flabobacterium, Kluyvere, Micobacterium, Haemophilus, Micoplana, Protaminobacter, Candida, Saccharomyces, Bacillus, thermophile Bacillus, Pseudomonas, Micrococcus, Hafnia, Proteus, Vibrio, Staphyrococcus, Propionibacterium, Sartina, Planococcus, Escherichia, Kurthia, Rhodococcus, Acinetobacter, Xanthobacter, Streptomyces, Rhizobium, Salmonella, Klebsiella, Enterobacter, Erwinia, Aeromonas, Citrobacter, Achromobacter, Agrobacterium, Arthrobacter and Pseudonocardia.

Recent advance in molecular biology and genetic engineering has made preparation of a genetically engineered recombinant strain possible and relatively easier, by obtaining a gene for a nucleoside phosphorylase from the above strain though analysis of the molecular-biological properties, the amino acid sequence and the like of the protein in the strain; constructing a recombinant plasmid into which the gene and a regulatory region for its expression is inserted; and introducing the plasmid into a given host to obtain the recombinant strain to allow the recombinant strain to express the protein. In the light of the recent technical level, such a genetically engineered recombinant strain obtained by introducing the gene for a nucleoside phosphorylase into a desired host may be included in the microorganisms capable of expressing the phosphorylase according to the present invention.

A regulatory region necessary for expression herein may be a promoter sequence (may include an operator sequence controlling transcription), a ribosome binding sequence (SD sequence), a transcription termination sequence, or the like. Examples of a promoter sequence include a trp operator in a tryptophane operon derived from E. coli; a lac promoter in a lactose operon; a $P_L$ promoter and a $P_R$ promoter derived from lambda phage; a gluconate synthase promoter (gnt) derived from Bacillus subtilis; an alkali protease promoter (apr); a neutral protease promoter (npr); and α-amylase promoter (amy). An uniquely modified and designed sequence such as a tac promoter may be used. A ribosome binding sequence may be, for example, a sequence derived from E. coli or Bacillus subtilis, but any sequence may be used as long as it can function in a desired host such as E. coli and Bacillus subtilis. For example, one can use a consensus sequence formed by DNA synthesis, that is, a sequence with more than 4 consecutive bases complementary to 3'-terminal region in 16S ribosome RNA. A transcription termination sequence is not always necessary, but a $\rho$-factor independent terminator such as a lipoprotein terminator and a trp operon terminator may be used. Desirably, these regulatory regions on a recombinant plasmid may be sequentially aligned as follows; from upstream of 5'-terminal, a promoter sequence, a ribosome binding sequence, a nucleoside phosphorylase coding gene and a transcription termination sequence.

As concrete examples of a plasmid herein, pBR 322, pUC18, Bluescript II SK(+), pKK223-3 and pSC101 having an autonomously replicable region in E. coli; pUB110, pTZ4, pC194, $\rho$11, $\phi$1 and $\phi$105 having an autonomously replicable region in Bacillus subtilis may be used as a vector. As examples of a plasmid autonomously replicable in two or more hosts, pHV14, TRp7, YEp7 and pBS7 may be used as a vector.

A given host herein may be typically, but not limited to, Escherichia coli as described in Examples later, but other strains such as Bacillus sp. including Bacillus subtilis, as well as yeasts and actinomyces may be used.

The nucleoside phosphorylase or the microorganism capable of expressing its activity in this invention may be a commercially available enzyme, a microorganism strain expressing its activity, a processed material of the strain or an immobilized product thereof. A processed material of the strain may be, for example, acetone-dried cells or bacterial debris prepared by an appropriate procedure such as mechanical destruction, ultrasonic disintegration, freezing and thawing, pressurization and depressurization, osmotic pressure methods, autolysis, cell-wall decomposition and surfactant treatment. If necessary, the strain may be further purified by ammonium sulfate precipitation, acetone precipitation or column chromatography.

In this invention, a metal cation capable of forming a water-insoluble salt with phosphate ion may be, without restriction, any metal cation which can form a water-insoluble salt with phosphate ion as a byproduct in the reaction for precipitation. Examples of such metal cation include calcium, magnesium, barium, iron, cobalt, nickel, copper, silver, molybdenum, lead, zinc and lithium ions. Among these, particularly preferable are industrially universal and safe metal ions which do not adversely affect the reaction, e.g., calcium, barium and aluminum ions.

The metal cation capable of forming a water-insoluble salt with phosphate ion in this invention may be added in the reaction solution as a salt(s) of a metal cation(s) capable of forming a water-insoluble salt(s) with phosphate ion with at least one anion selected from chloride, nitride, carbonate, sulfate and acetate ions. Examples of such a salt include calcium chloride, calcium nitride, calcium carbonate, calcium sulfate, calcium acetate, barium chloride, barium nitride, barium carbonate, barium sulfate, barium acetate, aluminum chloride, aluminum nitride, aluminum carbonate, aluminum sulfate and aluminum acetate.

A metal cation in this invention may be present as a salt with a pentose-1-phosphate in the reaction solution. Concrete examples of such salt include ribose-1-phosphate calcium salt, 2-deoxyribose-1-phosphate calcium salt, 2,3-dideoxyribose-1-phosphate calcium salt, arabinose-1-phosphate calcium salt, ribose-1-phosphate barium salt, 2-deoxyribose-1-phosphate barium salt, 2,3-dideoxyribose-1-phosphate barium salt, arabinose-1-phosphate barium salt, ribose-1-phosphate aluminum salt, 2-deoxyribose-1-phosphate aluminum salt, 2,3-dideoxyribose-1-phosphate aluminum salt and arabinose-1-phosphate aluminum salt.

An aqueous reaction medium constituting a reaction solution used in this invention is a reaction medium mainly comprising water. Any of such media may be used as long as it can eliminate phosphate ion from the reaction system as an insoluble salt precipitate by reacting it with a metal cation while the desired reaction utilizing enzyme activity in this invention proceeds. Examples of such an aqueous reaction medium include water and aqueous reaction media prepared by adding a water-soluble organic solvent such as a lower alcohol, acetone and dimethylsulfoxide to water. When using a water-soluble organic solvent, a rate of the water-soluble organic solvent to water may be, for example, 0.1 to 70 wt%. For ensuring stability of the reaction, a reaction solution comprising the aqueous reaction medium may be prepared as a buffer. Known buffers, which can be used for the enzyme reaction for this invention, may be used as the buffer.

A reaction for preparing a nucleoside compound in this invention may be conducted under the conditions such as appropriate pH and temperature, depending on a target nucleoside, a pentose-1-phosphate and a nucleic acid base or its analogue as substrates, a nucleoside phosphorylase or a microorganism exhibiting the activity of the enzyme or one of various preparations from the microorganism as a reaction catalyst, and the type and the properties of a metal salt added for eliminating phosphoric acid from the reaction system. The reaction may be generally carried out at a pH in the range of 5 to 10 and a temperature in the range of 10 to 60 °C. If pH is not within the control range, it may be possible that the reaction conversion rate reduces due to, for example, poor stability of the target product or substrate, reduction in enzyme activity and failure to forming the water-insoluble salt with phosphoric acid. If pH varies in the course of the reaction, an acid such as hydrochloric acid and sulfuric acid or an alkali such as sodium hydroxide and potassium hydroxide may be added at an appropriate timing, if necessary. The concentrations of a pentose-1-phosphate and a nucleic acid base or its analogue are suitably about 0.1 to 1000 mM. In terms of a molar ratio between them, a molar ratio of a nucleic acid base or its analogue to a pentose-1-phosphate or its salt may be 0.1 to 10, preferably 0.95 or less in the light of a reaction conversion rate. When using two or more of nucleic acid bases or their analogues as a mixture, the above concentration and molar ratio may be selected for the total amount used.

A metal cation capable of forming a water-insoluble salt with phosphoric acid to be added may be added in a molar ratio of 0.1 to 10, more preferably 0.5 to 5 to a pentose-1-phosphate used in the reaction. In a reaction with a higher concentration, a nucleic acid base or its analogue as a substrate or a nucleoside as a product may be not be completely dissolved in the reaction solution. This invention may be also applied to such a case. When using a mixture of two or more materials as a metal cation, the above molar ratio may be selected for the total amount used.

A nucleoside compound thus produced may be isolated from the reaction solution by a common procedure such as concentration, crystallization, dissolution, electrodialysis and adsorption and desorption using an ion-exchange resin or charcoal.

Examples

This invention will be explained with reference to, but not limited to, Examples and Comparative Examples.

Analytical method

All nucleoside compounds prepared were assayed by high performance liquid chromatography under the following analysis conditions:

Column: YMC-Pack ODS-A312 150 $\times$ 6.0 mm I.D. (YMC Co., Ltd);
Column temperature: 40 °C;
Pump flow rate: 0.75 mL/min;
Detection: UV 260 nm;
Eluent: 10 mM phosphoric acid : acetonitrile = 95:5 (v/v).

Example 1

One mL of a reaction mixture consisting of 2.5 mM 2'-deoxyribose-1-phosphate di(monocyclohexylammonium) salt (SIGMA), 2.5 mM adenine (Wako Pure Chemicals, Extra Pure), 12 units/mL purine nucleoside phosphorylase (Funakoshi), 10 mM calcium chloride (Wako Pure Chemicals, Extra Pure) and 10 mM Tris hydrochloride buffer (pH 7.4) was reacted at 30 °C for 24 hours. At the end of the reaction, a white precipitate had been formed. After dilution, the reaction mixture was analyzed. The results indicated formation of 2.40 mM deoxyadenosine in an yield of 96.0 %.

Comparative Example 1

A reaction was conducted according to the same procedure as described in Example 1 except that calcium chloride was not added. At the end of the reaction, precipitation was not observed. After dilution, the reaction mixture was analyzed. The results indicated formation of 2.01 mM deoxyadenosine in an yield of 80.4 %.

Example 2

A reaction was conducted according to the same procedure as described in Example 1 except that a calcium chloride concentration was 2.5 mM. At the end of the reaction, a white precipitate had been formed. After dilution, the reaction mixture was analyzed. The results indicated formation of 2.27 mM deoxyadenosine in an yield of 90.8 %.

Example 3

A reaction was conducted according to the same procedure as described in Example 1 except that aluminum chloride was added for calcium chloride. At the end of the reaction, a white precipitate had been formed. After dilution, the reaction mixture was analyzed. The results indicated formation of 2.31 mM deoxyadenosine in an yield of 93.3 %.

Example 4

A reaction was conducted according to the same procedure as described in Example 1 except that barium chloride was added for calcium chloride. At the end of the reaction, a white precipitate had been formed. After dilution, the reaction mixture was analyzed. The results indicated formation of 2.31 mM deoxyadenosine in an yield of 92.4 %.

Example 5

A reaction was conducted according to the same procedure as described in Example 4 except that the barium chloride concentration was 2.5 mM. At the end of the reaction, a white precipitate had been formed. After dilution, the reaction mixture was analyzed. The results indicated formation of 2.27 mM deoxyadenosine in an yield of 90.2 %.

Example 6

As described in Journal of Biological Chemistry, Vol.184, pp. 449-459 (1950), 2-deoxyribose-1-phosphate barium salt was prepared. One mL of a reaction mixture consisting of 2.5 mM 2-deoxyribose-1-phosphate barium salt, 2.5 mM adenine (Wako Pure Chemicals, Extra Pure), 12 units/mL purine nucleoside phosphorylase (Funakoshi), 10 mM calcium chloride (Wako Pure Chemicals, Extra Pure) and 10 mM Tris hydrochloride buffer (pH 7.4) was reacted at 30 °C for 24 hours. At the end of the reaction, a white precipitate had been formed. After dilution, the reaction mixture was analyzed. The results indicated formation of 2.40 mM deoxyadenosine in an yield of 91.1 %.

Example 7

One mL of a reaction mixture consisting of 2.5 mM 2-deoxyribose-1-phosphate di(monocyclohexylammonium) salt (SIGMA), 2.5 mM thymine (Wako Pure Chemicals, Extra Pure), 12 units/mL thymidine phosphorylase (SIGMA), 10 mM calcium nitrate (Wako Pure Chemicals, Extra Pure) and 10 mM Tris hydrochloride buffer (pH 7.4) was reacted at 30 °C for 24 hours. At the end of the reaction, a white precipitate had been formed. After dilution, the reaction mixture was analyzed. The results indicated formation of 2.28 mM thymidine in an yield of 91.2 %.

Comparative Example 2

A reaction was conducted according to the same procedure as described in Example 7 except that calcium nitrate was not added. At the end of the reaction, precipitation was not observed. After dilution, the reaction mixture was analyzed. The results indicated formation of 1.88 mM thymidine in an yield of 75.2 %.

Example 8

One mL of a reaction mixture consisting of 2.5 mM ribose-1-phosphate cyclohexylammonium salt (SIGMA), 2.5 mM 2,6-diaminopurine (Aldrich), 12 units/mL purine nucleoside phosphorylase (Funakoshi), 10 mM calcium chloride (Wako Pure Chemicals, Extra Pure) and 10 mM Tris hydrochloride buffer (pH 7.4) was reacted at 30 °C for 24 hours. At the end of the reaction, a white precipitate had been formed. After dilution, the reaction mixture was analyzed. The results indicated formation of 1.94 mM 2,6-diaminopurine riboside in an yield of 77.6 %.

Comparative Example 3

A reaction was conducted according to the same procedure as described in Example 8 except that calcium chloride was not added. At the end of the reaction, precipitation was not observed. After dilution, the reaction mixture was analyzed. The results indicated formation of 1.54 mM 2,6-diaminopurine riboside in an yield of 61.6 %.

Example 9

E. coli chromosome DNA was prepared as follows.
Fifty mL of an LB medium was inoculated with Escherichia coli K-12/XL-10 strain (Stratagene Inc.) and it was cultured at 37 °C overnight. After collection, the bacteria was lysed with a lysis solution containing 1 mg/mL of lysozyme. The lysis solution was treated with phenol and DNA was precipitated as usual by ethanol precipitation. The DNA precipitate was collected with a glass rod and washed to prepare the desired DNA for PCR.
Oligonucletides of SEQ ID Nos. 1 and 2 (prepared by a contracted manufacturer, Hokkaido System Science Co. Ltd.) designed based on the sequence a known deoD gene in Escherichia coli (GenBank accession No. AE000508 with a coding region of base numbers 11531 to 12250) were used as primers for PCR. These primers have restriction enzyme recognition sequences for EcoRI and Hind III near 5'- and 3'-ends, respectively.

## SEQ ID No. 1: gtgaattcac aaaaaggata aaaca atg gc

## SEQ ID No. 2: tcgaagcttg cgaaacacaa tta ctc ttt

Using 0.1 mL of a PCR reaction solution containing 6 ng/µL of the above E. coli chromosome DNA, which had been completely digested by restriction enzyme Hind III, and the primers (each at 3µM), PCR was conducted by 30 cycles under the conditions of denaturation: 96 °C, 1 min; annealing: 55 °C, 1 min; elongation: 74 °C, 1 min per a cycle.

The above reaction product and a plasmid pUC18 (Takara Shuzo Co. Ltd.) were digested by EcoRI and Hind III and ligated using Ligation-High (Toyobo Co. Ltd.). The recombinant plasmid thus obtained was used to transform Escherichia coli DH5α. The transformed strain was cultured in an LB agar medium containing 50 µg/mL of ampicillin and X-Gal (5-bromo-4-chloro-3-indolyl-β-D-galactoside) to provide an Am-resistant transformant as a white colony.

A plasmid was extracted from the transformant thus obtained and the plasmid in which a desired DNA fragment had been inserted was designated as pUC-PNP73. The transformant thus obtained was designated as Escherichia coli MT-10905.

Escherichia coli MT-10905 was cultured by shaking at 37 °C overnight in 100 mL of an LB medium containing 50 µg/mL ampicillin. The culture medium was centrifuged at 13,000 rpm for 10 min to collect the cells. The cells were suspended in 10 mL of 10 mM Tris-hydrochloride buffer (pH 8.0) and ultrasonicated to give a homogenate to be used as an enzyme source.

One mL of a reaction mixture consisting of 10 mM 2'-deoxyribose-1-phosphate di(monocyclohexylammonium) salt (SIGMA), 10 mM adenine (Wako Pure Chemicals, Extra Pure), 20 mM calcium chloride, 100 mM Tris hydrochloride buffer (pH 8.0) and 20 units/mL the purine nucleoside phosphorylase described above was reacted at 50 °C for 20 hours.

At the end of the reaction, a white precipitate had been formed. After dilution, the reaction mixture was analyzed. The results indicated formation of 9.6 mM deoxyadenosine in an yield of 96.0 %.

Comparative Example 4

A reaction was conducted according to the same procedure as described in Example 9 except that calcium chloride was not added. At the end of the reaction, precipitation was not observed. After dilution, the reaction mixture was analyzed. The results indicated formation of 7.9 mM deoxyadenosine in an yield of 79 %.

Example 10

One mL of a reaction mixture consisting of 10 mM 2'-deoxyribose-1-phosphate di(monocyclohexylammonium) salt (SIGMA), 10 mM guanine (Wako Pure Chemicals, Extra Pure), 20 mM calcium chloride, 100 mM Tris hydrochloride buffer (pH 8.0) and 20 units/mL the purine nucleoside phosphorylase prepared as described in Example 9 was reacted at 50 °C for 20 hours.

At the end of the reaction, a white precipitate had been formed. After dilution, the reaction mixture was analyzed. The results indicated formation of 8.0 mM deoxyguanosine in an yield of 80.0 %.

Comparative Example 5

A reaction was conducted according to the same procedure as described in Example 10 except that calcium chloride was not added. At the end of the reaction, precipitation was not observed. After dilution, the reaction mixture was analyzed. The results indicated formation of 5.1 mM deoxyguanosine in an yield of 51 %.

Example 11

One mL of a reaction mixture consisting of 10 mM 2'-deoxyribose-1-phosphate di(monocyclohexylammonium) salt (SIGMA), 10 mM thymine (Wako Pure Chemicals, Extra Pure), 20 mM calcium chloride, 100 mM Tris hydrochloride buffer (pH 8.0) and 20 units/mL thymidine phosphorylase was reacted at 50 °C for 20 hours.

At the end of the reaction, a white precipitate had been formed. After dilution, the reaction mixture was analyzed. The results indicated formation of 9.0 mM thymidine in an yield of 90.0 %.

Comparative Example 6

A reaction was conducted according to the same procedure as described in Example 11 except that calcium chloride was added. At the end of the reaction, precipitation was not observed. After dilution, the reaction mixture was analyzed. The results indicated formation of 7.0 mM thymidine in an yield of 70 %.

Industrial Applicability

According to this invention, in preparation of a nucleoside compound from a pentose-1-phosphate and a nucleic acid base using nucleoside phosphorylase or a microorganism exhibiting activity of the enzyme, a metal salt capable of forming a water-insoluble salt with phosphoric acid may be added to eliminate phosphoric acid as byproduct from a reaction system. This addition leads to an improved yield which have not been achieved according to the prior art technique. In terms of industrial practice, this invention leads to considerable cost reduction and is thus quite significant.

**Claims**

1. A process for producing a nucleoside compound comprising the step of reacting a pentose-1-phosphate with a nucleic acid base or an analogue of the nucleic acid base in the presence of nucleoside phosphorylase activity in an aqueous reaction medium to form the nucleoside compound,
   which is **characterized in** allowing a metal cation capable of forming a water-insoluble salt with phosphate ion to be present in the aqueous reaction medium as a metal salt with one or more than one anion selected from the group consisting of chloride, nitride, carbonate, sulfate and acetate ions.

2. A process for producing a nucleoside compound as claimed in Claim 1, wherein the pentose-1-phosphate is ribose-1-phosphate or 2-deoxyribose-1-phosphate.

3. A process for producing a nucleoside compound as claimed in Claim 1 or 2, wherein the metal cation capable of forming a water-insoluble salt with phosphoric ion is one ion or more than one ion selected from the group consisting of calcium, barium and aluminum ions.

4. A process for producing a nucleoside compound as claimed in Claim 3, wherein the metal cation capable of forming a water-insoluble salt with phosphoric ion is present as a pentose-1-phosphate salt in the aqueous reaction medium.

**Patentansprüche**

1. Verfahren zur Herstellung einer Nukleosidverbindung, umfassend den Schritt des Umsetzens eines Pentose-1-phosphats mit einer Nukleinsäurebase oder einem Analog der Nukleinsäurebase in Gegenwart von Nukleosidphosphorylaseaktivität in einem wässrigen Reaktionsmedium zur Bildung der Nukleosidverbindung,
   **dadurch gekennzeichnet, dass** ein Metallkation, das mit einem Phosphation ein wasserunlösliches Salz bilden kann, im wässrigen Reaktionsmedium als Metallsalz mit einem oder mehr als einem Anion, ausgewählt aus der Gruppe bestehend aus Chlorid-, Nitrid-, Carbonat-, Sulfat- und Acetationen, vorhanden ist.

2. Verfahren zur Herstellung einer Nukleosidverbindung gemäss Anspruch 1, worin das Pentose-1-phosphat Ribose-1-phosphat oder 2-Desoxyribose-1-phosphat ist.

3. Verfahren zur Herstellung einer Nukleosidverbindung gemäss Anspruch 1 oder 2, worin das Metallkation, das mit einem Phosphorsäureion ein wasserunlösliches Salz bilden kann, ein Ion oder mehr als ein Ion ist, ausgewählt aus der Gruppe bestehend aus Calcium-, Barium- und Aluminiumionen.

4. Verfahren zur Herstellung einer Nukleosidverbindung gemäss Anspruch 3, worin das Metallkation, das mit einem Phosphorsäureion ein wasserunlösliches Salz bilden kann, als ein Pentose-1-Phosphatsalz im wässrigen Reaktionsmedium vorhanden ist.

**Revendications**

1. Procédé de production d'un composé nucléoside, qui comprend l'étape consistant à faire réagir un pentose-1-phosphate avec une base d'acide nucléique ou un analogue de base d'acide nucléique en présence d'une activité de nucléoside phosphorylase dans un milieu réactionnel aqueux pour former un composé nucléoside,
   qui est **caractérisé en ce qu'**un cation métallique capable de former un sel insoluble dans l'eau avec un ion phosphate est présent dans le milieu réactionnel aqueux en tant que sel métallique ayant un ou plusieurs anions choisis dans le groupe consistant en les ions chlorure, nitrure, carbonate, sulfate, et acétate.

2. Procédé de production d'un composé nucléoside selon la revendication 1, dans lequel le pentose-1-phosphate est le ribose-1-phosphate ou le 2-désoxyribose-1-phosphate.

3. Procédé de production d'un composé nucléoside selon la revendication 1 ou la revendication 2, dans lequel le cation métallique capable de former un sel insoluble dans l'eau avec l'ion phosphorique est constitué d'un ou plusieurs ions choisis dans le groupe consistant en les ions calcium, baryum et aluminium.

4. Procédé de production d'un composé nucléoside selon la revendication 3, dans lequel le cation métallique capable de former un sel insoluble dans l'eau avec l'ion phosphorique est présent sous forme d'un pentose-1-phosphate dans le milieu réactionnel aqueux.